# EUROPÄISCHE PATENTANMELDUNG

(11) **EP 0 673 922 A1**
(43) Veröffentlichungstag der Anmeldung: **27.09.1995**
(21) Anmeldenummer: 95500034.4
(22) Anmeldetag: 23.03.1995
(51) Int. Cl.: C07C 237/46, A61K 49/04

(54) **Neue nicht ionische iodhaltige Dimeren als Kontrastmittel für Röntgenbilder, Verfahren zu ihrer Herstellung und pharmazeutische Zusammensetzungen, die sie enthalten**

(30) Priorität: 25.03.1994 WO PCT/ES94/00033
(71) Anmelder: CENTRO INVESTIGACION JUSTESA IMAGEN S.A., E-28028 Madrid (ES)
(72) Erfinder: Martin Jimenez, José L., E-28220 Majadahonda (Madrid) (ES); Carretero Colon, José Ma, E-28030 Madrid (ES); Bohle, Florian, E-28034 Madrid (ES); Alonso Silva, Ignacio, E-28017 Madrid (ES); Harto Martinez, Juan R., E-28012 Madrid (ES); Gonzalez Tavares, Leonor, E-28029 Madrid (ES); Garrido, Perez, Mercedes, D-28028 Madrid (ES)
(74) Vertreter: Lehmann Novo, Maria Isabel

(57) **Zusammenfassung**

Die vorliegende Erfindung betrifft neue nicht ionische iodhaltige Kontrastmittel für Röntgenstrahlung, die einen dimeren Aufbau und die allgemeine Formel I aufweisen, bei denen die Bindungsbrücke durch ein Derivat der Hydroxymalonsäure oder Cetomalonsäure gebildet wird 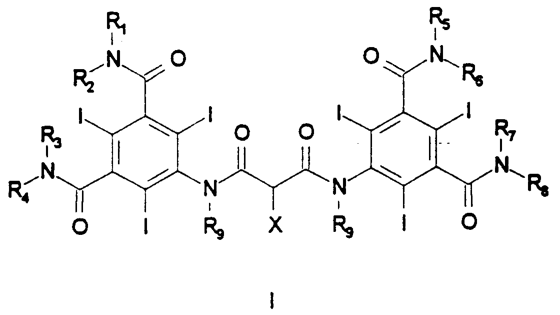
worin
- R₁ bis R₈ gleich oder verschieden sind und Wasserstoff, Methyl oder einen geradkettigen oder verzweigten Polyhydroxy-(C₂-C₄-alkyl)-Rest bedeuten;
- R₉ Wasserstoff, Methyl oder einen geradkettigen oder verzweigten Polyhydroxy- (C₂-C₄-alkyl)-Rest bedeutet;
- X Wasserstoff, einen Hydroxyl-Rest (-OH) oder eine Oxo-Gruppe (=O) bedeutet.

Die Erfindung betrifft auch ein Verfahren zur Herstellung der bevorzugten Verbindungen der allgemeinen Formel I und die pharmazeutischen Präparate, die diese Verbindungen enthalten und die für die Röntgendiagnose bestimmt sind.

## Beschreibung

Die vorliegende Erfindung betrifft eine neue Gruppe von nicht ionischen dimeren Derivaten der 5-Amino-triiod-isophthalamid- und der 5-Amino-triiodisophthalsäure, die in ihrem Molekül Brücken von Malonsäure, Hydroxymalonsäure (Tartronsäure) und Cetomalonsäure (Mesoxalsäure) einschließen und die der allgemeinen Formel I entsprechen: 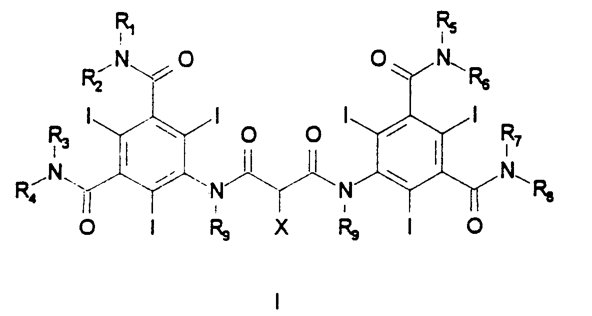
worin
- R₁ bis R₈: gleich oder verschieden sind und Wasserstoff, Methyl oder einen Polyhydroxy-alkyl-Rest bedeuten, worin Alkyl für einen geradkettigen oder verzweigten C₂-C₄-Rest steht;
- R₉: Wasserstoff, Methyl oder einen geradkettigen oder verzweigten Polyhydroxy-(C₂-C₄-alkyl)-Rest bedeutet;
- X: Wasserstoff, einen Hydroxyl-Rest (-OH) oder eine Oxo-Gruppe (=O) bedeutet.

Davon werden die Verbindungen gemäß den Beispielen 1 bis 14 bevorzugt, von denen in der Tabelle I die Bedeutungen der Gruppen A₁ bis R₉ und X zusammengefaßt werden und deren chemisch-physikalische und toxikologische Daten in der Tabelle II wiedergegeben werden. Solche Verbindungen gehören zur Gruppe der sogenannten nicht ionischen iodhaltigen Dimeren und werden als Kontrastmittel für Röntgenuntersuchungen eingesetzt, die nach intravaskulärer Gabe das Entstehen von Bildern der Organe bzw. Körpergebilde ermöglichen. Solche Produkte sollen nicht nur die Salze der 2,4,6-Triiodbenzoesäure und der 2,4,6-Triiodisophthalsäure sondern auch die nicht ionischen monomeren Derivate dieser Säuren ersetzen, die heutzutage für diesen Zweck in Einsatz sind. Grund für den Austausch: eine bessere Verträglichkeit, weil einige der neuen Produkte einen gleichwertigen Gehalt an Iod haben und dadurch gleiche Röntgenopazität erzeugen, gleichzeitig aber weisen sie einen in wäßriger Lösung niedrigeren osmotischen Druck und somit eine niedrigere Osmolalität auf und dieser ist ein ganz wichtiger Faktor, der für die ungünstigen Reaktionen, wie Schmerz bzw. Hitzeempfindung, und für kardiovaskuläre Störungen verantwortlich ist. Solche Reaktionen entstehen nach der Verabreichung der Kontrastlösung. Erfolgt die Verabreichung für die myelographische bzw. zisternographische Technik, so erlebt man außerdem Konvulsionen (Krämpfe) und epileptogene Störungen, weil die wäßrige Lösungen, z.B. Lösungen von Salzen der 2,4,6-Triiodbenzoesäure oder der 2,4,6-Triiodisophthalsäure bzw. von nicht ionischen Monomeren, die in den subarachnoidalen Raum injiziert werden, eine hohe Osmolalität haben müssen, die sich zwischen 600 und 2.000 mOs/kg bewegt, um die für die diagnostische Wirksamkeit nötige Iodkonzentration von 300 mg/ml zu erreichen.

Aus diesem Grund brauchen die Röntgenologen neue Verbindungen, bei denen solche Schwierigkeiten nicht auftreten und die für ein breites Spektrum von Röntgentechniken nutzbar sind, wie zum Beispiel für die Angiographie, Urographie, Myelographie, Zisternographie, Kardioangiographie, Lymphographie, Gastrographie, usw. und die als wäßrigen Lösungen herstellbar sind, die mit dem Blut und mit dem Liquor cerebrospinalis isotonisch sind.

Dieser Bedarf hat zur Entwicklung einer neuen Reihe von Verbindung der allgemeinen Formel I geführt, bei denen die Osmolalität in wäßriger Lösung stark herabgesetzt ist, bis hin zu hypotonischen Lösungen, die aber für die Diagnose noch ausreichende Iodkonzentrationen aufweisen.

Diese Lösungen haben aber in der Regel eine höhere Viskosität als die ionischen Verbindungen oder als die nicht ionischen Monomere; einige der neuen Verbindungen sind sogar in wäßriger Lösung nicht stabil genug, da sie zur Bildung von übersättigten Lösungen neigen. Aus diesem Grund hat sich unsere Forschung auf die Suche nach hexaiodierten dimeren Derivaten orientiert, die durch eine hohe Löslichkeit in Wasser, eine mäßige Viskosität, eine niedrige Osmolalität sowie eine hohe intravenöse, intrazerebrale und intrazisternale Verträglichkeit gekennzeichnet sind. Diese Produkte sind gerade Gegenstand der vorliegenden Erfindung.

Die Produkte der Erfindung der allgemeinen Formel I sind durch eine gute Löslichkeit in Wasser bei 20°C, eine Osmolalität in wässriger Lösung, die kleiner als die Osmolalität des Blutes (280 mOs/kg) ist, sowie durch eine niedrige allgemeine Toxizität gekennzeichnet, die als mittlere wirksame Dosis (LD₅₀) in Ratte bei intravenöser Gabe ausgedrückt wird.

In der Tabelle II werden die den bevorzugten Verbindungen der vorliegenden Erfindung entsprechenden Daten im Vergleich mit einer anderen bekannten Verbindung, dem Iodixanol, zusammengestellt. Iodixanol hat auch den Aufbau eines nicht ionischen hexaiodierten Dimere (s. EP 108 638, der Firma Nyeegard and Co.).

Die Verbindungen gemäß der vorliegenden Erfindung sind als Opazitätsmittel für Röntgenstrahlung bei Konzentrationen zwischen 150 und 500 mg I/ml und bei Dosierungen zwischen 10 und 250 ml, je nach der verwendeten Technik, einsetzbar.

**Tabelle I.**

| Bevorzugte Substituenten | |
|---|---|
| Bsp. Nr. | Substituenten |
| 1 | R₁=R₃=R₅=R₇= H; R₂=R₆= CH₃; R₄=R₈=CH₂CHOHCH₂OH; R₉= CH₂CH₂OH; X = OH |
| 2 | R₁=R₃=R₅=R₇= H; R₂=R₆ =CH₃; R₄=R₈=R₉= CH₂CHOHCH₂OH; X = OH |
| 3 | R₁=R₃=R₅=R₇= H; R₄=R₈= CH₂CHOHCH₂OH; R₂=R₆=R₉=CH₂CH₂OH; X = H |
| 4 | R₁=R₃=R₅=R₇= H; R₄=R₈=R₉ CH₂CHOHCH₂OH; R₂=R₆=CH₂CH₂OH, X = H |
| 5 | R₁=R₂=R₃=R₅=R₆=R₇= H; R₄=R₈= CH₂CHOHCH₂OH; R₉= CH₃; X = OH |
| 6 | R₁=R₂=R₃=R₅=R₆=R₇= H; R₄=R₈= CH(CH₂OH)CHOHCH₂OH; R₉= CH₃; X = OH |
| 7 | R₁=R₂=R₃=R₅=R₇= H; R₄=R₆=R₈= CH₂CHOHCH₂OH; R₉= CH₃; X = OH |
| 8 | R₁=R₂=R₃=R₅=R₆=R₇= H; R₄=R₈= CH₂CHOHCH₂OH; R₉= CH₂CH₂OH; X = OH |
| 9 | R₁=R₂=R₃=R₅=R₆=R₇= H; R₄=R₈= CH(CH₂OH)CHOHCH₂OH; R₉= CH₂CH₂OH; X = OH |
| 10 | R₁=R₃=R₅=R₇= H; R₄=R₈= CH₂CHOHCH₂OH; R₂=R₆=R₉= CH₂CH₂OH; X = OH |
| 11 | R₁=R₃=R₅=R₇= H; R₄=R₈=R₉= CH₂CHOHCH₂OH; R₂=R₆= CH₂CH₂OH; X = OH |
| 12 | R₁=R₂=R₃=R₅=R₆=R₇= H; R₄=R₈=R₉= CH₂CHOHCH₂OH; X = OH |
| 13 | R₁=R₃=R₅=R₇= H; R₂=R₄=R₆=R₈= CH₂CHOHCH₂OH; R₉= CH₃; X = = O |
| 14 | R₁=R₂=R₃=R₅=R₆=R₇= H; R₄=R₈= CH₂CHOHCH₂OH; R₉= CH₃; X = =O |

**Tabelle II**

| Bp. Nr. | **Verbindung** | **% I** | **Löslichkeit** in Wasser bei 20°C und 300 mg I/ml | **Viskosität** bei 37°C und 300 mg I/ml (mPa·s) | **Osmolatität** bei 37°C und 300 mg I/ml (mOs/kg) | **LD**₅₀ Ratte (g I/kg) |
|---|---|---|---|---|---|---|
| 1 | ICJ-2993 | 53,7 | <60% | -- | -- | -- |
| 2 | ICJ-3093 | 50,0 | <60% | -- | -- | -- |
| 3 | ICJ-3593 | 50,6 | >60% | 7,5 | 160 | >15 |
| 4 | ICJ-3693 | 48,6 | >60% | 6,8 | 190 | >15 |
| 5 | ICJ-2293 | 55,4 | <60% | -- | -- | -- |
| 6 | ICJ-2693 | 53,1 | <60% | -- | -- | -- |
| 7 | ICJ-2493 | 52,6 | >60% | 8,1 | 170 | >15 |
| 8 | ICJ-3193 | 53,1 | <60% | -- | -- | -- |
| 9 | ICJ-3293 | 51,0 | <60% | -- | -- | -- |
| 10 | ICJ-3793 | 50,0 | >60% | 7,3 | 170 | >15 |
| 11 | ICJ-3893 | 48,1 | >60% | 7,8 | 210 | >15 |
| 12 | ICJ-2393 | 51,0 | >60% | 6,5 | 240 | >15 |
| 13 | ICJ-2893 | 50,1 | >60% | 10,9 | 160 | >15 |
| 14 | ICJ-3993 | 55,5 | <60% | -- | -- | -- |
| IODIXANOL | | 49,1 | >60% | 8,7 | 200 | >15 |

Diese wäßrigen Lösungen können zusammen mit pharmazeutisch annehmbaren Mitteln, wie Komplexbildner für Schwermetalle, z.B. mit Natrium- bzw. Calcium-Salzen der Ethylendiamintetraessigsäure (EDTA), in Konzentrationen, die zwischen 0,05 und 2 mM/l liegen, mit pH-Stabilisatoren, wie Tromethamin (= Trometamol) oder Phosphat-, Citrat- bzw. Bicarbonat-Ionen sowie als ionische Lösungen formuliert werden, die physiologisch mit Cl⁻, Ca²⁺, Mg²⁺, Na⁺, K⁺, usw. ausbalanciert sind, um wäßrige Lösungen der Verbindungen der allgemeinen Formel I zu erzielen, die isoosmolär mit den biologischen Flüssigkeiten sind.

Gemäß einer bevorzugten Methode werden die Verbindungen der Formel I durch N-Alkylierung von Malonamiden der allgemeinen Formel II hergestellt:

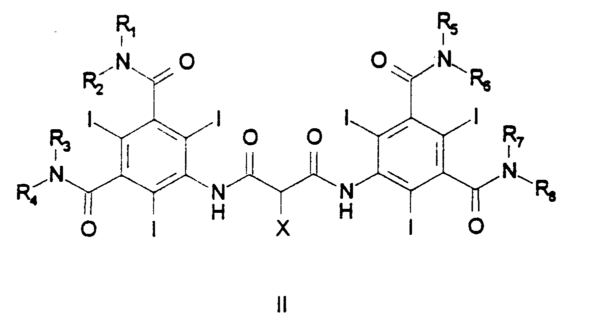

Die N-Alkylierung erfolgt durch ein Alkylierungsmittel der Formel

A-R₉

in Gegenwart von einem alkalischen Katalysator, wobei die Reste von R₁ bis R₉ und die Gruppe X die oben angegebenen Bedeutungen haben und die Gruppe A ein Halogenatom, eine Sulfat- oder Alkylsulfat-Gruppe o ein anderer Substituent ist, der fähig ist, eine N-Alkylierungsreaktion zu bewerkstelligen.

Während der Alkylierung können die OH-Gruppen, die in den Resten R₁ bis R₉ vorliegen, können, frei sein oder geschützt werden, wie Acetate, Isopropylidenderivate oder Dioxepine, in Abhängigkeit von ihrer Anzahl und Lage. Solche Schutzgruppen werden danach in einfacher Weise durch die herkömmlichen Methoden der Chemie entfernt, wie zum Beispiel durch Hydrolyse.

Die Verbindungen der allgemeinen Formel II werden aus den Säurechloridderivanten der Formel III bzw. IV hergestellt 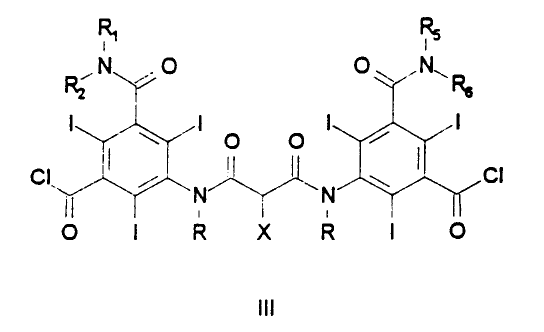 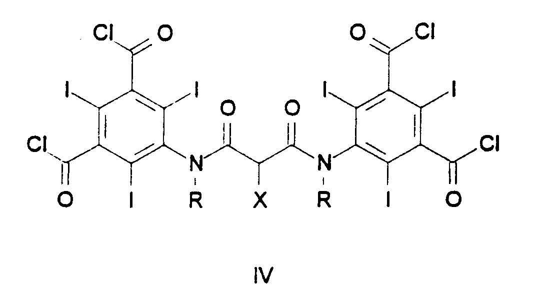
und zwar durch Umsetzung mit einem Amin der Formel H₂N-B in Gegenwart von einem alkalischen Katalysator, wobei R₁, R₂, R₅, R₆ und X die oben genannte Bedeutung haben und R Wasserstoff oder eine Methyl-Gruppe ist und B ein geradkettiger oder verzweigter Polyhydroxy-(C₂-C₄-alkyl)-Rest ist, worin die OH-Gruppen frei oder wie Acetate, Isopropylidenderivate oder Dioxepine geschützt sein können, je nach ihrer Anzahl und Lage.

Die Verbindungen der Formel III oder IV werden durch Umsetzung des 5-Amino-(oder -Methylamino-)-2,4,6-triiodisophthalamidoylchlorids, des 5-Amino-(oder -Methylamino-)-2,4,6-triiod-N-methyl-isophthalamidoylchlorids oder des 5-Amino-(oder -Methylamino-)-2,4,6-triiodphthaloylchlorids mit dem Malonsäurechlorid und ihren Derivaten der allgemeinen Formel V hergestellt 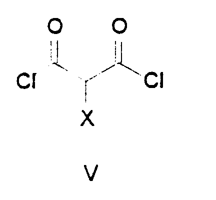
worin X die oben angegebene Bedeutung hat.

Die Endverbindungen der allgemeinen Formel I werden durch Behandlung mit Ionenaustauschharzen und mit anderen herkömmlichen Reinigunsmethoden gereinigt, oder aber durch Gas-Flüssig-Chromatographie (GLC).

Anschließend wird die Darstellung der bevorzugten Verbindungen beschrieben, die in der Tabelle I genannt werden.

### Beispiel 1

N,N-Bis-(2-hydroxyethyl)-N,N'-bis-(3-[N-(2,3-dihydroxypropyl)carbamoyl]-5-(N-methylcarbamoyl)-2,4,6-triiodphenyl}hydroxymalonamid (ICJ-2993)
A) 5-Amino-N-methyl-2,4,6-triiodisophthalamidoyl-chlorid
   Eine Mischung aus 120 g (0,20 Mol) 5-Amino-2,4,6-triiodisophthaloyl-chlorid, 21,3 g (0,20 Mol) wasserfreiem Natriumcarbonat und 19,1 g (0,22 Mol) wäßrigem Methylamin (40 %ig) in 600 ml Ethylacetat wird in einem geschlossenen Behälter bei Raumtemperatur während 24 Stunden gerührt. Der gebildete Feststoff wird filtriert, mit Wasser gewaschen und in Vakuum getrocknet.
   Ausbeute: 65,4 g (55 %).
B) N,N'-Bis-(3-chlorcarbonyl-5-(N-methylcarbamoyl)-2,4,6-triiodphenyl)-acetoxymalonamid
   59,0 g (0,10 Mol) der Verbindung gemäß dem Beispiel 1A werden in 180 ml trockenes Dioxan gelöst. Die Lösung wird auf 40-45°C erhitzt und es werden 9,9 g (0,05 Mol) Acetoxymalonylchlorid, gelöst in 20 ml trockenem Dioxan, hinzugefügt. Nach dieser Zugabe wird die Mischung auf 80°C während 24 Stunden erhitzt. Man läßt sie auf Raumtemperatur abkühlen und man filtriert und trocknet den gebildeten Feststoff.
C) N,N'-Bis-{3-[N-(2,3-(isopropylidendioxy)propyl)carbamoyl]-5-(N-methylcarbamoyl)-2,4,6-triiodphenyl}acetoxymalonamid
   Einer Mischung aus 26,1 dg (0,020 Mol) der im Beispiel 1B beschriebenen Verbindung und 6,4 g (0,060 Mol) wasserfreiem Natriumcarbonat in 125 ml Dimethylformamid werden 10,0 g (0,060 Mol) 2,2-Dimethyl-5-aminomethyl-1,3-dioxolan-Hydrochlorid zugegeben und die Mischung wird auf 40°C während 20 Stunden erhitzt. Man läßt sie auf Raumtemperatur abkühlen und filtriert die unlöslichen Salze, die sich in Aufschwemmung befinden. Das Filtrat wird im Vakuum bis zur Trockne werdampft und der feste Rückstand wird mit Diethylether zerrieben.
   Ausbeute: 26,7 g (85 %).
D) N,N'-Bis-(2-hydroxyethyl)-N,N'-bis-{3-[N-(2,3-dihydroxypropyl)carbamoyl]-5-(N-methylcarbamoyl)-2,4,6-triiodphenyl}-hydroxymalonamid
   Es wird eine Aufschlämmung aus 23,5 g (0,015 Mol) der im Beispiel 1C beschriebenen Verbindung und 28,5 g (0,082 Mol) Natriumphosphat-Dodekahydrat in 110 ml Methanol vorbereitet. Sie wird auf 50°C erhitzt und es werden 5 ml (0,075 Mol) 2-Chlorethanol hinzugefügt. Man läßt sie bei dieser Temperatur während 48 Stunden stehen, man filtriert die unlöslichen Salze, die sich in Aufschwemmung befinden. Die Lösung wird zur Trock verdampft. Der gebildete Rückstand wird in Wasser/Methanol aufgeschlämmt, auf 40°C erhitzt und bei einem pH-Wert zwischen 1 und 1,5 durch Zugabe von 5 %ig. Salzsäure 24 Stunden lang gehalten, danach wird der pH-Wert auf 11-11,5 durch Zugabe von 5 %ig. Natronlauge eingestellt und 24 Stunden lang bei der genannten Temperatur gehalten. Man kühlt es auf Raumtemperatur ab, man stellt den pH-Wert neutral ein und die gebildete Lösung wird zur Trockne eingedampft. Der Rückstand wird durch Umkehrphase-GLC gereinigt, wobei Gemische aus Methanol und Wasser als Elutionsmittel eingesetzt werden.
   Ausbeute: 12,9 g (59 %).

### Beispiel 2

N,N'-Bis-(2,3-dihydroxypropyl)-N,N'-bis-{3-[N-(2,3-dihydroxypropyl)carbamoyl]-5-(N-methylcarbamoyl)-2,4,6-triiodphenyl}-hydroxymalonamid (ICJ-3093)

Es wird eine Aufschlämmung aus 23,5 g (0,015 Mol) der im Beispiel 1C beschriebenen Verbindung und 28,5 g (0,082 Mol) Natriumphosphat-Dodekahydrat in 110 ml Methanol vorbereitet. Sie wird auf 50°C erhitzt und es werden 6,3 ml (0,075 Mol) 3-Chlor-1,2-propandiol hinzugefügt. Man läßt sie bei dieser Temperatur während 48 Stunden stehen, man filtriert die unlöslichen Salze, die sich in Aufschwemmung befinden. Die methanolische Lösung wird zur Trockne eingedampft. Der gebildete Rückstand wird in Wasser/Methanol aufgeschlämmt, auf 40°C erhitzt und bei einem pH-Wert zwischen 1 und 1,5 durch Zugabe von 5 %ig. Salzsäure 24 Stunden lang gehalten, danach wird der pH-Wert auf 11-11,5 durch Zugabe von 5 %ig. Natronlauge eingestellt und 24 Stunden lang bei der genannten Temperatur gehalten. Man kühlt es auf Raumtemperatur ab, man stellt den pH-Wert neutral ein und die gebildete Lösung wird zur Trockenheit eingedampft. Der Rückstand wird durch Umkehrphase-GLC gereinigt, wobei Gemische aus Methanol und Wasser als Elutionsmittel eingesetzt werden.

Ausbeute: 14,2 g (62 %).

### Beispiel 3

N,N'-Bis-(2-hydroxyethyl)-N,N'-bis-{3-[N-(2,3-dihydroxypropyl)-carbamoyl]-5-[N-(2-hydroxyethyl)carbamoyl]-2,4,6-triiodphenyl}-malonamid (ICJ-3593)
A) N,N'-Bis-{3-chlorcarbonyl-5-[N-(2,3-diacetoxypropyl)carbamoyl]-2,4,6-triiodphenil}malonamid
   Zu einer auf 80-90 °C erhitzten Lösung aus 110,2g (0,150 Mol) 5-Amino-N-(2,3-diacetoxypropyl)-2,4,6-triiodisophthalamidoyl-chlorid in 240 ml trockenem Dioxan werden mit kräftigem Rühren 8,8 ml (12,7 g; 0,090 Mol) Malonylchlorid gegeben. Die Reaktionsmischung wird während 4-6 Stunden weiter gerührt, abgekühlt, das Lösemittel wird in Vakuum verdampft und der gebildete Rückstand wird mit Toluol zerrieben. Der erhaltene Feststoff wird filtriert, gewaschen und im Vakuum getrocknet.
   Ausbeute: 93,4 g (81 %)
B) N,N'-Bis-{3-[N-(2-hydroxyethyl)carbamoyl]-5-[N-(2,3-diacetoxypropyl)carbamoyl]-2,4,6-triiodphenyl}malonamid
   Ein Gemisch aus 45,0 g (0,029 Mol) der im Beispiel 5A beschriebenen Verbindung, 15,5 g (0,146 Mol) wasserfreiem Natriumcarbonat und 4,4 ml (4,5 g; 0,073 Mol) Ethanolamin in 270 ml Ethylacetat wird bei Siedetemperatur unter Rückfluß 48 Stunden lang erhitzt. Der gebildete Feststoff wird abfiltriert, von den anorganischen Salzen durch Waschen mit Wasser befreit und im Vakuum getrocknet.
   Ausbeute: 39,0 g (84 %).
C) N,N'-Bis-(2-hydroxyethyl)-N,N'-bis-{3-[N-(2-hydroxypropyl)carbamoyl]-5-[N-(2-hydroxyethyl)carbamoyl]-2,4,6-triiodphenyl}malonamid
   Zu einem Gemisch aus 39,0 g (0,025 Mol) der im Beispiel 5B beschriebenen Verbindung und 45,0 g (0,118 Mol) Trinatriumphosphat-Dodekahydrat in 200 ml Methanol werden 7,2 ml (0,108 Mol) 2-Chlorethanol gegeben, es wird während 2 Tage bei 50°C gerührt. Es wird abgekühlt, die Salze werden abfiltriert und das Filtrat wird bei vermindertem Druck eingedampft. Der Rückstand wird durch Auflösung in 150 ml Methanol und 150 ml Wasser und durch Behandlung mit wäßriger Natronlauge bei einem pH-Wert = 11,5 bei 50°C während 5 Stunden hydrolysiert. Der pH-Wert wird neutral eingestellt, die Mischung wird unter vermindertem Druck eingedampft und der gebildete Rückstand wird durch Umkehrphase-GLC gereinigt, wobei Gemische aus Methanol und Wasser als Elutionsmittel verwendet werden.
   Ausbeute: 19,6 g (52 %)

### Beispiel 4

N,N'-Bis-(2,3-hydroxypropyl)-N,N'-bis-{3-[N-(2,3-dihydroxypropyl)carbamoyl]-5-[N-(2-hydroxyethyl)carbamoyl]-2,4,6-triiodphenyl}malonamid (ICJ-3693)

Zu einem Gemisch aus 39,0 g (0,025 Mol) der im Beispiel 5B beschriebenen Verbindung und 45,0 g (0,118 Mol) Trinatriumphosphat-Dodekahydrat in 200 ml Methanol werden 9,0 ml (0,108 Mol) 3-Chlor-1,2-propandiol gegeben, es wird während 2 Tagen bei 50°C gerührt. Es wird abgekühlt, die Salze werden abfiltriert und das Filtrat wird bei vermindertem Druck eingedampft. Der Rückstand wird durch Auflösung in 150 ml Methanol und 150 ml Wasser und durch Behandlung mit wäßriger Natronlauge bei einem pH-Wert = 11,5 bei 50°C während 5 Stunden hydrolysiert. Der pH-Wert wird neutral eingestellt, die Mischung wird unter vermindertem Druck eindedampft und der gebildete Rückstand wird durch Umkehrphase-GLC gereinigt, wobei Gemische aus Methanol und Wasser als Elutionsmittel verwendet werden.

Ausbeute: 21,9 g (56 %)

### Beispiel 5

N,N'-Dimethyl-N,N'-bis-{5-carbamoyl-3-[N-(2,3-dihydroxypropyl)carbamoyl]-2,4,6-triiodphenyl}hydroxymalonamid (ICJ-2293)
A) 5-Methylamino-2,4,6-triiodisophthalamidoyl-chlorid
   100 g (0,164 Mol) 5-Methylamino-2,4,6-triiodisophthalamidoyl-chlorid werden in 200 ml Ethylacetat gelöst. Dazu werden 17,4 g (0,164 Mol) wasserfreies Natriumcarbonat und 12,2 ml (0,164 Mol) wäßriges Ammoniak (25 %ig) zugegeben. Die Mischung wird 24 Stunden lang bei Raumtemperatur gerührt. Der ausgeschiedene Feststoff wird abfiltriert, gewaschen und getrocknet.
   Ausbeute: 75 g ( 75 %).
B) N,N'-Dimethyl-N,N'-bis-(5-carbamoyl-3-chlorcarbonyl-2,4,6-triiodphenyl)acetoxymalonamid
   Zu 60 g (0,1 Mol) der im Beispiel 5A beschriebenen Verbindung, gelöst in 180 ml trockenem Dioxan, werden bei 80°C 10,1 g (0,05 Mol) Acetoxymalonyl-chlorid, verdünnt in 10 ml trockenes Dioxan, zugegeben. Diese Temperatur wird 18 Stunden lang gehalten. Man läßt die Mischung auf Raumtemperatur abkühlen, Dioxan wird bis zur Trockne verdampft. Der gebildete Rückstand wird in Ethylacetat gelöst, mit 5 %ig. Natriumbicarbonat und mit Wasser gewaschen und zur Trockenheit eingedampft.
   Ausbeute: 50,2 g (75 %).
C) N,N'-Dimethyl-N,N'-bis-{5-carbamoyl-3-[N-(2,3-dihydroxypropyl)carbamoyl]-2,4,6-triiodphenyl}hydroxymalonamid
   Zu einer Mischung aus 32,4 g (0,025 Mol) der im Beispiel 5B beschriebenen Verbindung und 2,1 g (0,08 Mol) wasserfreies Natriumcarbonat in 240 ml Ethylacetat werden 13,4 g (0,08 Mol) 2,2-Dimethyl-5-aminomethyl-1,3-dioxolan-Hydrochlorid zugegeben, die Mischung wird auf Siedetemperatur erhitzt und unter Rückfluß 72 Stunden lang gehalten. Der sich in Aufschwemmung befindende Feststoff wird abfiltriert, mit Wasser gewaschen und getrocknet. Das erhaltene Produkt (35,2 g) wird in Wasser/Methanol aufgeschlämmt, bei 40°C gehalten und mit Salzsäure (5 %ig.) auf einen pH-Wert von 1 bis 1,5 während 24 Stunden gebracht und danach mit 5 %ig. Natronlauge auf einen pH-Wert von 11 bis 11,5 während 24 Stunden bei gleicher Temperatur gebracht. Es wird auf Raumtemperatur abgekühlt, auf neutralen pH-Wert gebracht und die erhaltene Lösung wird zur Trockenheit eingedampft. Der Rückstand wird durch Umkehrphase-GLC gereinigt, wobei Gemische aus Methanol und Wasser als Elutionsmittel verwendet werden.
   Ausbeute: 18,3 g (53 %).

### Beispiel 6

N,N'-Dimethyl-N,N'-bis-{5-carbamoyl-3-[N-(1-hydroxymethyl-2,3-dihydroxypropyl)carbamoyl]-2,4,6-triiodphenyl}-hydroxymalonamid (ICJ-2693)

Zu einer Mischung aus 37,0 g (0,028 Mol) der im Beispiel 5B beschriebenen Verbindung und 24,3 g (0,084 Mol) Natriumcarbonat-Dodekahydrat in 185 ml Aceton werden 13,7 g (0,084 Mol) 6-Amino-2,2-dimethyl-5-hydroxy-1,2-dioxepin zugegeben. Die Mischung wird auf Siedetemperatur erhitzt und unter Rückfluß 48 Stunden lang gehalten. Sie wird auf Raumtemperatur abgekühlt, die Salze werden abfiltriert und das Filtrat wird zur Trockenheit eingedampft. Der erhaltene Rückstand wird in Wasser/Methanol aufgeschlämmt, bei 40°C gehalten und mit Salzsäure (5 %ig.) auf einen pH-Wert von 1 bis 1,5 während 24 Stunden gebracht und danach mit 5 %ig. Natronlauge auf einen pH-Wert von 11 bis 11,5 während 24 Stunden bei gleicher Temperatur gebracht. Er wird auf Raumtemperatur abgekühlt, auf neutralen pH-Wert gebracht und die erhaltene Lösung wird zur Trockenheit eingedampft. Der Rückstand wird durch Umkehrphase-GLC gereinigt, wobei Gemische aus Methanol und Wasser als Elutionsmittel verwendet werden.

Ausbeute: 15,5 g (40 %).

### Beispiel 7

N,N'-Dimethyl-N-{5-carbamoyl-3-[N-(2,3-dihydroxypropyl)carbamoyl]-2,4,6-triiodphenyl}-N'-{3,5-bis-[N-(2,3-dihydroxypropyl)carbamoyl]-2,4,6-triiodphenyl}hydroxymalonamid (ICJ-2493)
A) N-Methyl-N-[3,5-bis-(chlorcarbonyl)-2,4,6-triiodphenyl)acetoxymalonamidoyl-chlorid
   Zur einer Aufschlämmung aus 67,2 g (0.11 Mol) 5-Methylamino-2,4,6-triiodisophthaloyl-chlorid in 270 ml trockenem Dioxan werden 23,2 g (0,11 Mol) Acetoxymalonyl-chlorid, verdünnt in 50 ml trockenem Dioxan, gegeben. Sie wird bei Raumtemperatur 48 Stunden lang gerührt, das Lösemittel wird zur Trockne verdampft. Der erhaltene Feststoff wird in 50 ml Ethylacetat aufgeschlämmt, filtriert und getrocknet.
   Ausbeute: 70,0 g (82 %).
B) N,N'-Dimethyl-N-(5-carbamoyl-3-chlorcarbonyl-2,4,6-triiodphenyl)-N'-[3,5-bis-(chlorcarbonyl)-2,4,6-triiodphenyl]acetoxymalonamid
   Ein Gemisch aus 70,0 g (0,09 Mol) der im Beispiel 7A beschriebenen Verbindung und 53,5 g (0,09 Mol) der im Beispiel 5A beschriebenen Verbindung, gelöst in 370 ml trockenem Dioxan, wird auf 80-85°C erhitzt und bei dieser Temperatur während 48 Stunden gehalten. Es wird auf Raumtemperatur abgekühlt, das Lösemittel wird bis zur Trockenheit verdampft. Der Rückstand wird in Ethylacetat gelöst, mit 5 %ig. Natriumbicarbonat, mit 20 %ig. Natriumbisulphit und mit Wasser gewaschen. Die organische Phase wird bis zur Trockne verdampft und der Rückstand wird mit Diethyleter zerrieben.
   Ausbeute: 76,6 g (64 %).
C) N,N'-Dimethyl-N-{5-carbamoyl-3-[N-(2,3-dihydroxypropyl)carbamoyl]-2,4,6-triiodphenyl}-N'-{3,5-bis-[N-(2,3-dihydroxypropyl)carbamoyl]-2,4,6-triiodphenyl}hydroxymalonamid
   Zu einer Mischung aus 76,6 g (0,057 Mol) der im Beispiel 7B beschriebenen Verbindung und 24,4 g (0,23 Mol) wasserfreiem Natriumcarbonat werden 38,7 g (0,23 Mol) 2,2-Dimethyl-5-aminomethyl-1,3-dioxolan-Hydrochlorid zugegeben, die Mischung wird auf Siedetemperatur erhitzt und unter Rückfluß 72 Stunden lang gehalten. Sie wird abgekühlt, der sich in Aufschwemmung befindende Feststoff wird filtriert, mit Wasser gewaschen und getrocknet. Der erhaltene Feststoff wird in Wasser/Methanol aufgeschlämmt, bei 40°C gehalten und mit Salzsäure (5 %ig.) auf einen pH-Wert von 1 bis 1,5 während 24 Stunden gebracht und danach mit 5 %ig. Natronlauge auf einen pH-Wert von 11 bis 11,5 während 24 Stunden bei gleicher Temperatur gebracht. Es wird auf Raumtemperatur abgekühlt, auf neutralen pH-Wert gebracht und die erhaltene Lösung wird zur Trockne eingedampft. Der Rückstand wird durch Umkehrphase-GLC gereinigt, wobei Gemische aus Methanol und Wasser als Elutionsmittel verwendet werden.
   Ausbeute: 24,7 g (30 %).

### Beispiel 8

N,N'-Bis-(2-hydroxyethyl)-N,N'-bis-{5-carbamoyl-3-[N-(2,3-dihydroxypropyl)carbamoyl]-2,4,6-triiodphenyl}-hydroxymalonamid (ICJ-3193)

Methode 1: Eine Mischung aus 29,8 g (0,05 Mol) 5-Amino-2,4,6-triiodisophthaloyl-chlorid, 5,3 g (0,05 Mol) wasserfreiem Natriumcarbonat und 4,2 g (0,05 Mol) wäßrigem Ammoniak (25 %ig) in 150 ml Ethylacetat wird bei Raumtemperatur während 24 Stunden gerührt. Der gebildete Feststoff wird filtriert, mit Wasser gewaschen und im Vakuum getrocknet.

Ausbeute: 15,4 g (53 %).

Methode 2: Zu einer Aufschlämmung aus 53,0 g (0,1 Mol) 5-Amino-2,4,6-triiodisophthalamidsäure in 265 ml Ethylacetat werden 21,9 ml (0,3 Mol) Thionylchlorid gegeben, die Mischung wird bei Siedetemperatur unter Rückfluß 4 Stunden lang gehalten. Nach Vollständigung der Reaktion wird der Überschuß an Thionylchlorid durch Zugabe con 5,4 ml Wasser zerstört, danach wird Ethylacetat abdestilliert, fast bis zur Trockenheit, die Mischung wird abgekühlt, der gebildete Feststoff wird filtriert, mit Ethylacetat gewaschen und im Vakuum getrocknet.

Ausbeute: 44,4 g (77 %).
B) N,N'-Bis-(5-carbamoyl-3-chlorcarbonyl-2,4,6-triiodphenyl)acetoxymalonamid
   52,0 g (0,09 Mol) der Verbindung gemäß dem Beispiel 8A werden in 160 ml trockenem Dioxan gelöst. Die Lösung wird auf 40-45°C erhitzt und es werden 9,9 g (0,05 Mol) Acetoxymalonylchlorid, gelöst in 20 ml trockenes Dioxan, hinzugefügt. Nach dieser Zugabe wird die Mischung auf 80°C während 24 Stunden erhitzt. Man läßt sie auf Raumtemperatur abkühlen und man filtriert und trocket den ausgeschiedenen Feststoff.
   Ausbeute: 23,1 g (40 %).
C) N,N'-Bis-{5-carbamoyl-3-[N-(2,3-(isopropylidendioxy)propyl)carbamoyl]-2,4,6-triiodphenyl}acetoxymalonamid
   Zu einer Mischung aus 23,0 g (0,018 Mol) der im Beispiel 8B beschriebenen Verbindung und 5,7 g (0,054 Mol) wasserfreiem Natriumcarbonat in 115 ml Dimethylformamid werden 9,0 g (0,054 Mol) 2,2-Dimethyl-5-aminomethyl-1,3-dioxolan-Hydrochlorid zugegeben und die Mischung wird auf 40°C während 20 Stunden erhitzt. Man läßt sie auf Raumtemperatur abkühlen und filtriert die unlöslichen Salze, die sich in Aufschwemmung befinden. Das Filtrat wird in Vakuum zur Trockne eingedampft und der feste Rückstand wird mit Diethylether zerrieben.
   Ausbeute: 22,3 g (86 %).
D) N,N'-Bis-(2-hydroxyethyl)-N,N'-bis-{5-carbamoyl-3-[N-(2,3-dihydroxypropyl)carbamoyl]-2,4,6-triiodphenyl}hydroxymalonamid
   Es wird eine Aufschlämmung aus 20,0 g (0,015 Mol) der im Beispiel 8C beschriebenen Verbindung und 28,5 g (0,082 Mol) Natriumphosphat-Dodekahydrat in 110 ml Methanol vorbereitet. Sie wird auf 50°C erhitzt und es werden 5 ml (0,075 Mol) 2-Chlorethanol zugegeben. Man läßt sie bei dieser Temperatur während 48 Stunden stehen, man filtriert die unlöslichen Salze, die sich in Aufschwemmung befinden. Die methanolische Lösung wird zur Trockne eingedampft. Der gebildete Rückstand wird in Wasser/Methanol aufgeschlämmt, auf 40°C erhitzt und bei einem pH-Wert zwischen 1 und 1,5 durch Zugabe von 5 %ig. Salzsäure 24 Stunden lang gehalten, danach wird der pH-Wert auf 11-11,5 durch Zugabe von 5 %ig. Natronlauge eingestellt und 24 Stunden lang bei der genannten Temperatur gehalten. Man kühlt es auf Raumtemperatur ab, man stellt den pH-Wert neutral ein und die gebildete Lösung wird zur Trockenheit eingedampft. Der Rückstand wird durch Umkehrphase-GLC gereinigt, wobei Gemische aus Methanol und Wasser als Elutionsmittel eingesetzt werden.
   Ausbeute: 13,2 g (61 %).

### Beispiel 9

N,N'-Bis-(2-hydroxyethyl)-N,N'-bis-{5-carbamoyl-3-[N-(1-hydroxymethyl-2,3-dihydroxy-propyl)carbamoyl]-2,4,-6-triiodphenyl}hydroxymalonamid (ICJ-3293)
A) N,N'-Bis-{5-carbamoyl-3-[N-(7-hydroxy-4,4-dimethyl-3,5-dioxepinyl)carbamoyl]-2,4,6-triiodphenyl}acetoxymalonamid
   Zu einer Mischung aus 27,6 g (0,02 Mol) der im Beispiel 8A beschriebenen Verbindung und 6,3 g (0,06 Mol) wasserfreiem Natriumcarbonat in 125 ml Dimethylformamid werden 9,6 g (0,06 Mol) 6-Amino-2,2-dimethyl-5-hydroxy-1,2-dioxepin gegeben und die Mischung wird auf 40°C während 20 Stunden erhitzt. Man läßt sie auf Raumtemperatur abkühlen und filtriert die unlöslichen Salze, die sich in Aufschwemmung befinden. Das Filtrat wird in Vakuum zur Trockne eingedampft und der feste Rückstand wird mit Diethylether zerrieben.
   Ausbeute: 25,5 g (83 %).
B) N,N'-Bis-(2-hydroxyethyl)-N,N'-bis-{5-carbamoyl-3-[N-(1-hydroxy-2,3-dihydroxypropyl)carbamoyl]-2,4,6-triiodphenyl}-hydroxymalonamid
   Zu einer auf 50°C erhitzten Mischung aus 25,0 g (0,016 Mol) der im Beispiel 9A beschriebenen Verbindung und 33,4 g (0,088 Mol) Natriumphosphat-Dodekahydrat in 125 ml Methanol werden 5,3 ml (0,08 Mol) 2-Chlorethanol gegeben. Man läßt sie bei dieser Temperatur während 48 Stunden stehen, man kühlt sie auf Raumtemperatur ab, man filtriert die unlöslichen Salze und die methanolische Lösung wird zur Trockne eingedampft. Der gebildete Rückstand wird in Wasser/Methanol aufgeschlämmt, auf 40°C erhitzt und bei einem pH-Wert zwischen 1 und 1,5 durch Zugabe von 5 %ig. Salzsäure 24 Stunden lang gehalten, danach wird der pH-Wert auf 11-11,5 durch Zugabe von 5 %ig. Natronlauge eingestellt und 24 Stunden lang bei der genannten Temperatur gehalten. Man kühlt es auf Raumtemperatur ab, man stellt den pH-Wert neutral ein und die gebildete Lösung wird zur Trockene eingedampft. Der Rückstand wird durch Umkehrphase-GLC gereinigt, wobei Gemische aus Methanol und Wasser als Elutionsmittel eingesetzt werden.
   Ausbeute: 11,5 g (48 %).

### Beispiel 10

N,N'-Bis-(2-hydroxyethyl)-N,N'-bis-{3-[N-(2,3-dihydroxypropyl)carbamoyl]-5-[N-(2-hydroxyethyl)carbamoyl]-2,4,6-triiodphenyl}hydroxymalonamid (ICJ-3793)
A) N,N'-Bis-{3-chlorcarbonyl-5-[N-(2-hydroxyethyl)carbamoyl]-2,4,6-triiodphenyl}acetoxymalonamid
   Zu einer auf 80 - 90°C erhiztten Lösung aus 110,2 g (0,150 Mol) 5-Amino-N-(2,3-diacetoxypropyl)-2,4,6-triiodisophthalamidoyl-chlorid in 240 ml trockenem Dioxan werden unter kräftigem Rühren 17,9 g (0,090 Mol) Acetoxymalonylchlorid gegeben. Die Mischung wird 4-6 Stunden lang weiter gerührt, das Lösemittel wird im Vakuum verdampft und der erhaltene Rückstand wird in Toluol zerrieben. Der gebildete Feststoff wird filtriert, gewaschen und im Vakuum getrocknet.
   Ausbeute: 90,8 g (78 %).
B) N,N'-Bis-{3-[N-(2-hydroxyethyl)carbamoyl]-5-[N-(2,3-diacetoxypropyl)carbamoyl]-2,4,6-triiodphenyl}acetoxymalonamid
   Ein Gemisch aus 45,0 g (0,029 Mol) der im Beispiel 10A beschriebenen Verbindung, 15,5 g (0,146 mol) wasserfreiem Natriumcarbonat und 4,4 ml (4,5 g; 0,073 Mol) Ethanolamin in 270 ml Ethylacetat wird auf Siedetemperatur erhitzt und 48 Stunden lang unter Rückfluß gehalten. Der gebildete Feststoff wird filtriert, mit Wasser gewaschen (um ihn von den anorganischen Salzen zu befreien) und im Vakuum getrocknet.
   Ausbeute: 37,1 g (80 %)
C) N,N'-Bis-(2-hydroxyethyl)-N,N'-bis-{3-[N-(2,3-dihydroxypropyl)carbamoyl]-5-[N-(2-hydroxyethyl)carbamoyl]-2,4,6-triiodphenyl}acetoxymalonamid
   Zu einer auf 50°C geheizten Mischung aus 37,0 g (0,023 Mol) der im Beispiel 10B beschriebenen Verbindung und 41,4 g (0,109 Mol) Trinatriumphosphat-Dodekahydrat in 200 ml Methanol werden 6,7 ml (0,099 Mol) 2-Chlorethanol zugegeben, sie wird unter Rühren 2 Tage lang gehalten. Man kühlt sie ab, man filtriert die Salze und das Filtrat wird bei vermindertem Druck eingedampft. Der Rückstand wird durch Auflösung in 150 ml Methanol und 150 ml Wasser hydrolysiert, danach wird der pH-Wert von 11,5 durch Zugabe von Natronlauge eingestellt und 5 Stunden lang bei 50°C gehalten. Man stellt den pH-Wert neutral ein und die Lösung wird zur Trockne eingedampft. Das Produkt wird durch Umkehrphase-GLC gereinigt, wobei Gemische aus Methanol und Wasser als Laufmittel eingesetzt werden.
   Ausbeute: 15,0 g (43 %).

### Beispiel 11

N,N'-Bis-(2,3-dihydroxypropyl)-N,N'-bis-{3-[N-(2,3-dihydroxypropyl)carbamoyl]-5-[N-(2-hydroxyethyl)carbamoyl]-2,4,6-triiodphenyl}hydroxymalonamid (ICJ-3893)

Zu einer auf 50°C erhitzten Mischung aus 37,0 g (0,023 Mol) der im Beispiel 10B beschriebenen Verbindung und 41,4 g (0,109 Mol) Trinatriumphosphat-Dodekahydrat in 200 ml Methanol werden 8,3 ml (0,099 Mol) 3-Chlor-1,2-propandiol gegeben, sie wird unter Rühren 2 Tage lang gehalten. Man kühlt sie ab, man filtriert die Salze und das Filtrat wird bei vermindertem Druck eingedampft. Der Rückstand wird durch Auflösung in 150 ml Methanol und 150 ml Wasser hydrolysiert, danach wird der pH-Wert von 11,5 durch Zugabe von Natronlauge eingestellt und 5 Stunden lang bei 50°C gehalten. Man stellt den pH-Wert neutral ein und die Lösung wird zur Trockne eingedampft. Das Produkt wird durch Umkehrphase-GLC gereinigt, wobei Gemische aus Methanol und Wasser als Elutionsmittel eingesetzt werden.

Ausbeute: 14,2 g (39 %).

### Beispiel 12

N,N'-Bis-(2,3-dihydroxypropyl)-N,N'-bis-{5-carbamoyl-3-[N-(2,3-dihydroxypropyl)carbamoyl]-2,4,6-triiodphenyl}-hydroxymalonamid (ICJ-2393)

Es wird eine Aufschlämmung aus 22,0 g (0,015 Mol) der im Beispiel 8C beschriebenen Verbindung und 28,5 g (0,082 Mol) Natriumphosphat-Dodekahydrat in 110 ml Methanol vorbereitet. Sie wird auf 50 °C erhitzt und es werden 6,3 ml (0,075 Mol) 3-Chlor-1,2-propandiol hinzugefügt. Man läßt sie bei dieser Temperatur während 48 Stunden stehen, man kühlt sie auf Raumtemperatur ab, man filtriert die unlöslichen Salze, die sich in Aufschwemmung befinden. Die methanolische Lösung wird zur Trockne eingeengt. Der gebildete Rückstand wird in Wasser/Methanol aufgeschlämmt, auf 40°C erhitzt und bei einem pH-Wert zwischen 1 und 1,5 durch Zugabe von 5 %ig. Salzsäure 24 Stunden lang gehalten, danach wird der pH-Wert auf 11-11,5 durch Zugabe von 5 %ig. Natronlauge eingestellt und 24 Stunden lang bei der genannten Temperatur gehalten. Man kühlt es auf Raumtemperatur ab, man stellt den pH-Wert neutral ein und die gebildete Lösung wird zur Trockene eingedampft. Der Rückstand wird durch Umkehrphase-GLC gereinigt, wobei Gemische aus Methanol und Wasser als Elutionsmittel eingesetzt werden.

Ausbeute: 12,6 g (56 %).

### Beispiel 13

N,N'-Dimethyl-N,N'-bis-{3,5-bis[N-(2,3-dihydroxypropyl)carbamoyl]-2,4,6-triiodphenyl}cetomalonamid (ICJ-2893)
A) Cetomalonylchlorid
   Zu einer Mischung aus 5 g (0,036 Mol) Cetomalonsäure und 9,97 ml (0,11 Mol) α,α-Dichlormethylether wird eine Spatelspitze Zinkchlorid gegeben und sie wird bei Raumtemperatur während 30 Minuten und bei 60°C während einer Stunde gerührt. Das Produkt wird im Vakuum destilliert.
   Ausbeute: 3,8 (56 %)
B) N,N'-Dimethyl-N,N'-bis-[2,3-bis-(chlorcarbonyl)-2,4,6-triiodphenyl]cetomalonamid
   Zu einer auf 60°C erhitzten Lösung aus 40 g (0,065 Mol) 5-Methylamino-2,4,6-triiodisophthaloyl-chlorid in 90 ml Dioxan werden 7,4 g (0,004 Mol) der im Beispiel 13A beschriebenen Verbindung gegeben. Sie wird auf 90 °C aufernitzt, 24 Stunden bei dieser Temperatur gehalten, sie wird auf Raumtemperatur abgekühlt, das Dioxan wird bis zur Trockne verdampft. Der Rückstand wird in Ethylacetat gelöst, mit 5 %ig. Natriumbicarbonat und mit Wasser gewaschen. Er wird zur Trockne eingedampft und der Rückstand aus Toluol umkristallisiert.
   Ausbeute: 33,9 g (80 %).
C) N,N'-Dimethyl-N,N'-bis-{3,5-bis-[N-(2,3-dihydroxypropyl)carbamoyl]-2,4,6-triiodphenyl}cetomalonamid
   Ein Gemisch aus 39 g (0,023 Mol) der im Beispiel 13B beschriebenen Verbindung, 16,8 g (0,10 Mol) 2,2-Dimethyl-5-aminomethyl-1,3-dioxolan-hydrochlorid und 19,6 g (0,10 Mol) Natriumcarbonat-Pentahydrat in 150 ml Ethylacetat wird auf 50°C erhitzt und bei dieser Temperatur 24 Stunden gehalten. Die unlöslichen Salze werden abfiltriert und die organische Phase wird mit Wasser gewaschen, getrocknet und eingedampft. Das erhaltene Produkt (39,1 g) wird in Wasser/Methanol aufgeschlämmt und bei 40°C auf pH zwischen 1 und 1,3 durch Zugabe von 5 %ig. Salzsäure gebracht und 24 Stunden lang gehalten. Die gebildete Lösung wird neutral eingestellt und zur Trockne eingeengt. Der erhaltene Rückstand wird durch Umkehrphase-GLC gereinigt, wobei Gemische aus Methanol und Wasser als Laufmittel eingesetzt werden.
   Ausbeute: 27.9 g (79 %).

### Beispiel 14

N,N'-Dimethyl-N,N'-bis-{5-carbamoyl-3-[N-(2,3-dihydroxypropyl)carbamoyl]-2,4,6-triiodphenyl}cetomalonamid (ICJ-3993)
A) N,N'-Dimethyl-N,N'-bis-(5-carbamoyl-3-chlorcarbonyl)-2,4,6-triiodphenyl)cetomalonamid
   Zu einer auf 60°C erhitzten Lösung aus 40 g (0,067 Mol) der im Beispiel 5A beschriebenen Verbindung, gelöst in 90 ml trockenem Dioxan, werden 6,3 g (0,033 Mol) der im Beispiel 13A beschriebenen Verbindung, gelöst in 30 ml trockenem Dioxan, zugegeben. Nach beendeter Zugabe wird sie auf 90°C erhitzt, 24 Stunden bei dieser Temperatur gehalten. Das Dioxan wird bis zur Trockene verdampft und der Rückstand wird in Ethylacetat gelöst, mit 5 %ig. Natriumbicarbonat und mit Wasser gewaschen, getrocknet und zur Trockne eingedampft. Der Rückstand wird aus Toluol umkristallisiert.
   Ausbeute: 35,0 g (80 %).
B) N,N'-Dimethyl-N,N'-bis-{5-carbamoyl-3-[N-(2,3-dihydroxypropyl)carbamoyl]-2,4,6-triiodphenyl}cetomalonamid
   Ein Gemisch aus 30 g (0,023 Mol) der im Beispiel 14A beschriebenen Verbindung, 8,55 g (0,51 Mol) 2,2-Dimethyl-5-aminomethyl-1,3-dioxolan-hydrochlorid und 9,6 g (0,051 Mol) Natriumcarbonat-Pentahydrat in 150 ml Ethylacetat wird auf Siedetemperatur erhitzt und bei dieser Temperatur unter Rückfluß 24 Stunden gehalten. Die unlöslichen Salze werden abfiltriert und die organische Phase wird zur Trockene eingedampft. Das erhaltene Produkt (34 g) wird in Wasser/Methanol aufgeschlämmt und bei 40°C auf pH zwischen 1 und 1,3 durch Zugabe von 5 %ig. Salzsäure gebracht und 24 Stunden lang gehalten. Die gebildete Lösung wird neutral eingestellt und zur Trockene eingeengt. Der erhaltene Rückstand wird durch Umkehrphase-GLC gereinigt, wobei Gemische aus Methanol und Wasser als Elutionsmittel eingesetzt werden.
   Ausbeute: 25,5 g (78 %).

## Patentansprüche

1. Neue nicht ionische dimere Verbindungen, die Derivaten der 5-Amino-triiodisophthalamidsäure und der 5-Aminotriiodisophthalsäure der allgemeinen Formel I sind 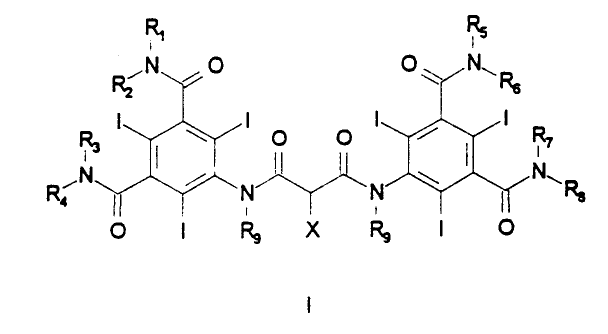 worin
- R₁ bis R₈ gleich oder verschieden sind und Wasserstoff, Methyl oder einen geradkettigen oder verzweigten Polyhydroxy-(C₂-C₄-alkyl)-Rest bedeuten;
- R₉ Wasserstoff, Methyl oder einen geradkettigen oder verzweigten Polyhydroxy-(C₂-C₄-alkyl)-Rest bedeutet;
- X Wasserstoff, einen Hydroxyl-Rest (-OH) oder eine Oxo-Gruppe (=O) bedeutet.

2. N,N-Bis-(2-hydroxyethyl)-N,N'-bis-{3-[N-(2,3-dihydroxypropyl)carbamoyl]-5-(N-methylcarbamoyl)-2,4,6-triiodphenyl}hydroxymalonamid.

3. N,N'-Bis-(2,3-dihydroxypropyl)-N,N'-bis-{3-[N-(2,3-dihydroxypropyl)carbamoyl]-5-(N-methylcarbamoyl)-2,4,6-triiodphenyl}hydroxymalonamid.

4. N,N'-Bis-(2-hydroxyethyl)-N,N'-bis-{3-[N-(2,3-dihydroxypropyl)-carbamoyl]-5-[N-(2-hydroxyethyl)carbamoyl]-2,4,6-triiodphenyl}-malonamid.

5. N,N'-Bis-(2,3-hydroxypropyl)-N,N'-bis-{3-[N-(2,3-dihydroxypropyl)carbamoyl]-5-[N-(2-hydroxyethyl)carbamoyl]-2,4,6-triiodphenyl}malonamid.

6. N,N'-Dimethyl-N,N'-bis-{5-carbamoyl-3-[N-(2,3-dihydroxypropyl)carbamoyl]-2,4,6-triiodphenyl}hydroxymalonamid.

7. N,N'-Dimethyl-N,N'-bis-{5-carbamoyl-3-[N-(1-hydroxymethyl-2,3-dihydroxypropyl)carbamoyl]-2,4,6-triiodphenyl}-hydroxymalonamid.

8. N,N'-Dimethyl-N-{5-carbamoyl-3-[N-(2,3-dihydroxypropyl)carbamoyl]-2,4,6-triiodphenyl}-N'-{3,5-bis-[N-(2,3-dihydroxypropyl)carbamoyl]-2,4,6-triiodphenyl}hydroxymalonamid.

9. N,N'-Bis-(2-hydroxyethyl)-N,N'-bis-{5-carbamoyl-3-[N-(2,3-dihydroxypropyl)carbamoyl]-2,4,6-triiodphenyl}-hydroxymalonamid.

10. N,N'-Bis-(2-hydroxyethyl)-N,N'-bis-{5-carbamoyl-3-[N-(1-hydroxymethyl-2,3-dihydroxy-propyl)carbamoyl]-2,4,-6-triiodphenyl}hydroxymalonamid.

11. N,N'-Bis-(2-hydroxyethyl)-N,N'-bis-{3-[N-(2,3-dihydroxypropyl)carbamoyl]-5-[N-(2-hydroxyethyl)carbamoyl]-2,4,6-triiodphenyl}hydroxymalonamid.

12. N,N'-Bis-(2,3-dihydroxypropyl)-N,N'-bis-{3-[N-(2,3-dihydroxypropyl)carbamoyl]-5-[N-(2-hydroxyethyl)carbamoyl]-2,4,6-triiodphenyl}hydroxymalonamid.

13. N,N'-Bis-(2,3-dihydroxypropyl)-N,N'-bis-{5-carbamoyl-3-[N-(2,3-dihydroxypropyl)carbamoyl]-2,4,6-triiodphenyl}-hydroxymalonamid.

14. N,N'-Dimethyl-N,N'-bis-(3,5-bis[N-(2,3-dihydroxypropyl)carbamoyl]-2,4,6-triiodphenyl}cetomalonamid.

15. N,N'-Dimethyl-N,N'-bis-{5-carbamoyl-3-[N-(2,3-dihydroxypropyl)carbamoyl]-2,4,6-triiodphenyl}cetomalonamid.

16. Pharmazeutische Zusammensetzungen, die für den Einsatz in Röntgenuntersuchung bestimmt sind und wenigstens eine der Verbindungen der Ansprüche 1 bis 15 umfassen, in wäßrigen Lösungen und in Iodkonzentrationen zwischen 100 und 400 mg pro ml und die auch pH-Wert-Stabilisatoren, wie den Puffer Trometamol (= Tris(hydroxymethyl)-aminomethan) sowie deren Salze, Phosphate, Citrate und Hydrogencarbonate, sowie steriles und apyrogenes Wasser enthalten können, und auch Komplexbildner für Schwermetalle, wie zum Beispiel das Natrium- und/oder Calciumsalz der Ethylendiamintetraessigsäure und andere pharmazeutisch annehmbare Komplexbildner enthalten können.

17. Methode zur Herstellung von Verbindungen der allgemeinen Formel I gemäß den Ansprüchen 1 bis 15, dadurch gekennzeichnet, daß man Malonamide der allgemeinen Formel II 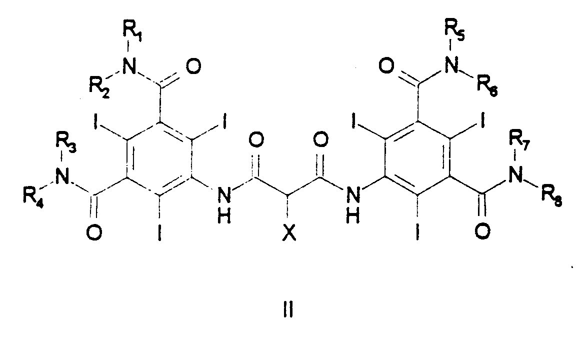 mit einem Alkylierungsmittel der Formel A-R₉ in Gegenwart eines alkalischen Katalisators umsetzt, wobei die Reste R₁ bis R₉ und die Gruppe X die oben angegebenen Bedeutungen haben und die Gruppe A ein Halogenatom, eine Sulfat- oder Alkylsulfatgruppe oder einen anderen Substituenten bedeutet, der fähig ist, eine N-Alkylierung zustandezubringen.
Im Laufe der Alkylierung können die in den Resten R₁ bis R₉ vorliegenden OH-Gruppen frei oder wie Acetate, Isopropyliden-derivate oder Dioxepine geschützt sein, je nach ihrer Anzahl und Lage. Solche Schutzgruppen sind danach einfach abzuspalten, z.B. durch die herkömmlichen Methoden der Chemie, wie durch Hydrolyse.
Die Verbindungen der allgemeinen Formel II werden aus den Derivaten von Säurechlorid der Formel III oder IV hergestellt 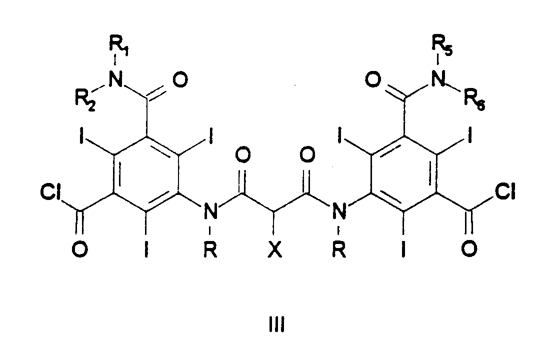 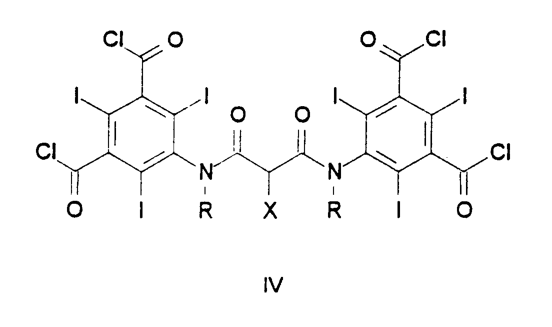 und zwar durch Umsetzung mit einem Amin der Formel H₂N-B in Gegenwart von einem alkalischen Katalysator, wobei R₁, R₂, R₅, R₆ und X die oben genannte Bedeutung haben und R Wasserstoff oder eine Methyl-Gruppe ist und B ein geradkettiger oder verzweigter Polyhydroxy-(C₂-C₄-alkyl)-Rest ist, worin die OH-Gruppen frei oder durch Acetate, Isopropyliden-derivate oder Dioxepine geschützt sein können, je nach ihrer Anzahl und Lage.
Die Verbindungen der Formel III oder IV werden durch Umsetzung des 5-Amino-(oder -Methylamino-)-2,4,6-triiodisophthalamidoylchlorids, des 5-Amino-(oder -Methylamino-)-2,4,6-triiod-N-methyl-isophthalamidoylchlorids oder des 5-Amino-(oder -Methylamino-)-2,4,6-triiodphthaloylchlorids mit dem Malonsäurechlorid und ihren Derivaten der allgemeinen Formel V hergestellt 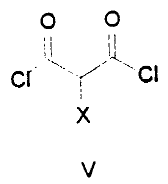 worin X die oben angegebene Bedeutung hat.
Die Endverbindungen der allgemeinen Formel I werden durch Behandlung mit Ionenaustauschharzen gereinigt, oder mit anderen herkömmlichen Reinigunsmethoden, oder aber durch Gas-Flüssig-Chromatographie (GLC).
